# FASCICULE DE BREVET EUROPEEN

(11) **EP 2 419 395 B1**
(45) Date de publication et mention de la délivrance du brevet: **10.07.2013**
(21) Numéro de dépôt: 10713610.3
(22) Date de dépôt: 31.03.2010
(51) Int. Cl.: C07C 51/09, C07C 59/08, C08F 8/50, C08F 8/12, C08F 6/28

(54) **RECYCLAGE CHIMIQUE DU PLA PAR HYDROLYSE**
CHEMISCHES RECYCLINGVERFAHREN VON PLA DURCH HYDROLYSIERUNG
CHEMICAL RECYCLING OF PLA BY HYDROLYSIS

(30) Priorité: 14.04.2009 BE 200900232
(43) Date de publication de la demande: 22.02.2012
(73) Titulaire: Galactic S.A., 7760 Escanaffles (BE)
(72) Inventeur: COSZACH, Philippe, B-7760 Escanaffles (BE); BOGAERT, Jean-Christophe, B-7760 Escanaffles (BE); WILLOCQ, Jonathan, B-7760 Escanaffles (BE)
(74) Mandataire: Decamps, Alain René François
(86) Numéro de dépôt international: PCT/EP2010/054274
(87) Numéro de publication internationale: WO 2010/118954

(56) Documents cités:
- EP-A1- 0 628 533
- WO-A1-92/00974
- US-A- 5 229 528

## Description

### Domaine de l'invention

La présente invention se rapporte à un procédé de recyclage chimique ou encore dénommé dépolymérisation du polylactide (PLA) , contenu ou non dans un mélange d'autres polymères, pour en reformer son monomère ou un dérivé de celui-ci.

A l'heure actuelle, afin de promouvoir l'essor des biopolymères, dont l'utilisation s'inscrit dans une perspective de respect de l'environnement, il est essentiel de pouvoir démontrer la viabilité de la gestion de la fin de vie de ces produits. L'objectif de la présente invention est de répondre à cette problématique pour le cas du polylactide (PLA) en proposant une solution originale se démarquant de celles déjà existantes.

### Etat de l'art

La gestion de la fin de vie des matières plastiques est un élément très important dans la viabilité d'un plastique sur le marché (par exemple, le PVC a été retiré du marché des bouteilles en plastique faute d'un système de recyclage efficace). Tout comme les plastiques d'origine non renouvelable (issus de la pétrochimie) et bien que leurs filières de fin de vie soient plus nombreuses, les biopolymères sont confrontés à des défis techniques lorsqu'il s'agit de cette gestion de fin de vie. Notamment lorsque l'on parle de volumes très importants, générés dans les marchés de commodité. C'est pourquoi, il est important de traiter ce problème.

A l'heure actuelle, différentes voies permettant de gérer la fin de vie des déchets sont déjà connues tels que la mise en décharge, l'incinération, le compostage, le recyclage mécanique ou encore le recyclage chimique.

Au niveau de la mise en décharge, il a été constaté que des polluants, principalement le méthane et le dioxyde de carbone, mais aussi des pesticides, des métaux lourds et des adjuvants, sont émis lors de la dégradation en décharge. Si mettre les déchets en décharge a longtemps été une solution pratique et peu coûteuse, il a été observé, outre les émissions de polluants précitées, que les déchets continuent de se dégrader produisant des lixiviats et des gaz qui doivent continuer à être évacués et traités pendant des périodes s'étendant jusqu'à plusieurs dizaines d'années. Dans le cas des biopolymères, toutefois, la pollution est moins importante étant donné que les produits de dégradation sont moins toxiques. Il faut malgré tout prendre en considération la durée de dégradation parfois assez longue, ce qui peut être problématique lorsque les volumes à traiter sont importants.

L'objectif de l'incinération est de réduire le volume de déchets en le convertissant en gaz (CO₂, H₂O, SOₓ, HCl, NOₓ, ...), il est donc inévitable que la composition de l'air au voisinage des incinérateurs soit altérée et contienne des niveaux de substances toxiques plus élevés. Dans le cas des bioplastiques, les rejets de CO₂ sont moins problématiques car le carbone n'est pas d'origine fossile, le bilan global reste donc neutre, voire légèrement positif en tenant compte des émissions dues au procédé (biomasse vers bioplastique). Par contre les autres rejets sont plus problématiques et entrainent donc inévitablement l'altération de la composition de l'air. Bien conçus et bien exploités, les incinérateurs pourraient réduire leurs émissions mais il s'agit d'une technologie extrêmement coûteuse tant en termes d'investissements que de dépenses. L'incinération offre cependant une alternative à la mise en décharge et permet une production d'énergie, en effet, une chaudière peut récupérer la chaleur et la valoriser, éventuellement sous forme d'énergie électrique et thermique (cogénération). Les incinérateurs ayant dans le passé été sources de pollutions importantes, ils ont été nommés « centres de valorisation thermique » puis « usines de valorisation énergétique» au lieu «d'usines d'incinération ». Cependant, les dossiers d'implantation de nouvelles unités sont de plus en plus complexes à gérer car les riverains n'acceptent plus d'avoir un incinérateur à proximité de leur domicile.

La biodégradabilité, propriété importante des biopolymères, peut être valorisée avantageusement par le compostage qui ne porte pas atteinte à l'environnement lorsque les précautions nécessaires sont prises, néanmoins, la progression du matériel de départ vers le stade final dépend d'un grand nombre de facteurs externes (dimensions des matériaux, taux d'humidité, aération, pH, flore bactérienne, ratio carbone-azote, ...) limitant parfois son utilisation. De plus, la difficulté à identifier et trier les produits contenant des polymères biodégradables (emballages alimentaires, sacs,...) peut détériorer la qualité du compost dans le cas où une erreur lors du tri serait effectuée. Par ailleurs, l'amélioration de la qualité du PLA (meilleure résistance thermique, meilleures propriétés mécaniques, ...) entraîne une dégradation plus lente.

Le recyclage mécanique est également connu et utilisé, dans le cas du polyéthylène téréphtalate (PET) par exemple. Il consiste à refondre la matière, seule ou en mélange avec de la matière vierge, pour fabriquer des produits commercialisables. Les déchets sont lavés, séchés, cristallisés et broyés puis directement transformés en produits finis ou bien en granulés qui peuvent alors être commercialisés. Cette voie est également applicable au PLA. Cependant les températures utilisées étant élevées, une dégradation du polymère est souvent observée, impliquant une perte de ses propriétés mécaniques et ce, pour le PLA comme pour tout autre polymère. Le produit peut dès lors être dirigé vers des applications moins nobles ou être mélangé à de la matière vierge. Ce type de recyclage n'est donc pas infini. De plus, le recyclage pose des problèmes lorsque les plastiques sont de compositions différentes puisqu'ils ne sont généralement pas compatibles entre eux. Les températures de transformation sont en effet différentes et le mélange de plusieurs plastiques entraîne une diminution de la qualité des caractéristiques mécaniques du produit final.

Ces différentes techniques de fin de vie ne sont pas idéales car les matières plastiques ne sont pas recyclées en éléments de base (monomères) et donc directement et perpétuellement utilisables. Malgré tout, ces procédés sont viables pour le PLA mais cela uniquement si le flux de matière est composé exclusivement de PLA. En effet, si d'autres polymères contaminent le PLA, les différentes techniques précitées sont rendues difficiles. Par exemple dans le cas d'une contamination au PET, ce dernier n'est pas dégradé dans un composte. Dans le cas d'une contamination au PVC, une incinération est possible mais implique l'utilisation de filtres coûteux en raison des dégagements nocifs. Quant au recyclage mécanique, le produit obtenu est complètement dénaturé s'il est composé d'un mélange de polymères.

Une autre voie de recyclage est également connue, le recyclage chimique. Souvent citée comme la voie idéale de recyclage, il consiste à transformer le polymère par un processus chimique tel que par exemple : le craquage thermique ou catalytique en hydrocarbure, la pyrolyse qui redonne les monomères, ... Un système de recyclage chimique pour le PET est connu, il s'agit de sa dépolymérisation par un diol, appelé aussi glycolyse. La chaîne moléculaire est rompue et les produits obtenus sont de l'acide téréphtalique et de l'éthylène glycol. Néanmoins, certains mécanismes de dégradation lors de cette dépolymérisation, engendrent des modifications structurales irréversibles de la matière, modifications qui peuvent être responsables de difficultés lors de transformations successives. Un système de recyclage chimique du PLA peut également être envisagé afin de récupérer le monomère, l'acide lactique ou l'un de ses dérivés. Certains brevets revendiquent par exemple, une hydrolyse rapide (Brake, L.D. ; Subramanian, N.S. U.S. Patent 5,229,528, 1993) ou une solvolyse (Brake, L.D. U.S. Patent 5,264,614, 1993 ; Brake, L.D. U.S. Patent 5,264,617, 1993) de poly(hydroxy-acide) incluant le PLA avec production d'hydroxy-acides ou de leurs esters. Ces procédés connus permettent même d'arriver à un rendement proche de 95% mais ceci implique de passer par de nombreuses étapes (une estérification suivie d'une distillation, ces étapes étant répétées à trois reprises). Il s'avère cependant qu'une telle manipulation présente un risque sérieux d'une prise en masse notamment lors des étapes de distillation, ce qui rend aléatoire une transposition du procédé à l'échelle industrielle. Il s'avère également que la mise en solution dans l'alcool n'est pas des plus faciles. En effet dans le cas de l'éthanol par exemple, il n'est pas possible d'ajouter de manière continue (et donc à pression atmosphérique) le PLA à une température supérieure à 78°C (point d'ébullition de l'éthanol). De par la faible densité de certains broyats non densifiés, cela a pour conséquence une concentration limitée en PLA. Par ailleurs, le PLA alimentant le flux du recyclage chimique contient généralement de l'eau en faible quantité. Cette eau pouvant provoquer une hydrolyse de l'ester formé, libérant de cette façon de l'acide lactique. En effet, la distillation ne pourra être menée de façon optimale, la présence de l'acide lactique favorisant une oligomérisation du milieu (BE Patent BE 20080424 « Procédé continu d'obtention d'un ester lactique »). Des dégradations thermiques (pyrolyse par exemple) du PLA sont également connues menant à la formation de lactide (F.D. Kopinke, M. Remmler, K. Mackenzie, M. Möder, O. Wachsen, Polymer Degradation and stability, 53, 329-342, 1996) par un mécanisme de cyclisation par addition-élimination. Mais ces méthodes présentent un faible rendement en monomères. De plus, ces différentes techniques sont souvent réalisées à haute température et/ou haute pression ce qui provoque une dégradation chimique et optique de l'acide lactique obtenu.

Le document WO92/00974 décrit un procédé de recyclage permettant de récupérer de l'acide lactique à partir de lactide contenant diverses impuretés. Ce procédé comprend la mise en solution du lactide pour l'isoler des impuretés insolubles puis son hydrolyse qui permet d'obtenir de l'acide lactique. Les documents US5229528 et EP0628533 décrivent des procédés équivalents.

Il existe donc un besoin pour un procédé simple, efficace et non-dénaturant de dépolymérisation du PLA afin de pouvoir le recycler sous la forme de son monomère de base ou l'un de ses dérivés.

### Brève description de l'invention :

La présente invention a pour objet un procédé de recyclage chimique ou de dépolymérisation du PLA, contenu ou non dans un mélanges d'autres polymères, en acide lactique ou un de ses dérivés, comme un sel d'acide lactique, par hydrolyse, en conditions douces, en produisant des monomères de haute qualité et à haut rendement, en augmentant la productivité, en diminuant les émissions de CO₂ et en réduisant le coût énergétique.

Un autre objet de l'invention est de dissoudre le PLA dans un solvant du PLA qui ne bloque pas sa dépolymérisation et qui n'impose pas d'étapes supplémentaires de purification.

La présente invention a également pour objet un procédé de recyclage chimique d'un mélange de polymères contenant nécessairement du PLA, selon lequel on met le mélange en solution dans un solvant du PLA pour séparer d'abord les impuretés solides telles que les autres polymères que le PLA qui ne se sont pas dissous, et ensuite on soumet la solution de PLA à une hydrolyse pour transformer le PLA en son monomère ou un dérivé de celui-ci.

Le procédé de la présente invention a aussi pour objet d'utiliser comme solvant pour la mise en solution du PLA, un ester lactique de manière améliorer la productivité du procédé, tout en ayant un impact minime sur toutes les étapes du procédé de recyclage chimique de l'acide polylactique.

### Description détaillée de l'invention :

La demanderesse a trouvé que la conduite d'un tel procédé de dépolymérisation pouvait être nettement améliorée si l'on effectuait au préalable une mise en solution du PLA ou du mélange de polymères contenant du PLA dans un ester lactique.

Le procédé de l'invention comprend successivement les étapes suivantes ; on effectue tout d'abord un broyage du PLA ou du mélange de polymères contenant du PLA, on utilise un ester lactique pour mettre le PLA en solution et simultanément séparer les impuretés solides telles que les autres polymères que le PLA qui ne sont pas dissous, ensuite on soumet la solution ainsi obtenue à une dépolymérisation par hydrolyse et finalement on purifie l'acide lactique ou l'un de ses dérivés obtenu de sorte à obtenir des produits répondant aux demandes spécifiques du marché traditionnel tel que les applications industrielles voir même la polymérisation du PLA.

### 1. Le broyage des déchets de PLA

Dans le cadre de l'invention présentée, les matières premières utilisées lors de ce recyclage chimique peuvent provenir de produits hors spécification dans les unités de production, de chutes de production dans les unités de transformation ainsi que de produits finis en fin de vie. On effectue d'abord le broyage du PLA ou du mélange de polymères contenant du PLA selon n'importe quelle technique connue de l'homme de l'art, comme par exemple le broyage par cisaillement, par impact, à sec ou sous eau. L'objectif de cette étape étant d'augmenter la surface spécifique des matériaux, de manière à obtenir un rapport poids/volume compris entre 0,05 et 1,4 t/m3, ce qui permet de faciliter les étapes de manutention et d'accélérer l'étape suivante de dissolution, rendant le procédé plus facilement industrialisable. Dans le cadre de l'invention, une ou plusieurs étapes de broyage peuvent être envisagées, leur nombre étant fonction du produit de départ mais également du coût de ces opérations et de la granulation finale visée. Il est également possible de pré ou post traiter ces flux de PLA ou de mélange de polymères contenant du PLA notamment en procédant à un lavage à l'eau ou tout autres solutions tel que par exemple solution de soude, potasse, détergente, ... D'autres traitements, comme un tri manuel ou encore une séparation automatique (magnétique par exemple) peuvent être envisagés, tout cela dans le but d'éliminer d'éventuels déchets qui pourraient altérer la qualité du produit final ou en compliquer la purification. Il est également évident que si les déchets de PLA ou de mélange de polymères contenant du PLA, à traiter ont une surface spécifique adéquate pour démarrer la mise en solution, on peut supprimer cette étape de broyage sans se départir du procédé de la présente invention.
Suite à cette étape de broyage, lorsqu'elle est réalisée, une étape de densification peut être envisagée afin de compacter la matière, ce qui améliorerait les étapes de manutention et de logistique.

### 2. La mise en solution du PLA ou du mélange de polymères contenant du PLA broyé

On met ensuite le mélange de polymères contenant du PLA, broyé ou non et compacté ou non, en solution avant l'étape de dépolymérisation. La mise en solution peut également être réalisée sans un broyage préalable si la forme du PLA ou du mélange de polymères contenant du PLA (rapport poids/volume) le permet. En effet, l'une des problématiques du traitement de ce type de flux est la différence de masse spécifique des différents matériaux retraités et ce même après l'étape de broyage. Bien que l'on sache qu'un avantage majeur de cette mise en solution est d'éliminer la problématique de la faible densité de matière à traiter (même lorsqu'une étape de densification est réalisée), conduisant donc à l'amélioration de la productivité par unité de volume. Il faut encore que le solvant utilisé ne soit pas gênant pour les étapes ultérieures.

Tout d'abord, ceci permet de séparer facilement les autres polymères que le PLA et de les récupérer pour un traitement spécifique, séparé et ultérieur.

Divers solvants sont connus pour mettre en solution le PLA tels que le benzène, le toluène, éther isopropylique, diclorométhane, chloroforme, chlorobenzène, Bien que ces solvants puissent parfaitement convenir pour réaliser la mise en solution du PLA, ils ne sont cependant pas recommandés vu le contexte écologique visé dans ce cas.

De manière surprenante, la Demanderesse a trouvé qu'en réalisant cette mise en solution du PLA dans un ester d'acide lactique, on pouvait éviter l'étape supplémentaire ultérieure de séparation sans diminuer le rendement en matière recyclée ou dépolymérisée. Il s'agit d'esters tels que du lactate de méthyle, du lactate d'éthyle, du lactate d'isopropyle, du lactate de butyle, du lactate d'hexyle, et plus généralement d'un alkyle ester d'acide lactique dont le radical alkyle a de 1 à 12 atomes de carbones. On a également trouvé que la mise en solution dans l'ester lactique avait l'avantage de pouvoir être réalisée à des températures plus importantes que celles atteintes lors de la solubilisation dans l'alcool dont cet ester est dérivé. En effet la température d'ébullition de l'ester est généralement plus haute que celle de l'alcool, ce qui permet de mettre plus de PLA en solution. De plus, cette dissolution du PLA dans l'ester lactique s'effectue assez rapidement.

La Demanderesse a maintenant trouvé qu'il était possible par cette manipulation de doubler la capacité volumique en PLA et donc la quantité de matière traitée. Cette dissolution peut être préalable ou simultanée à l'étape suivante et réalisée à différentes températures allant jusqu'à la température de fusion du PLA.

Dans le cas d'une contamination du flux de PLA par un autre polymère (PET, PE, PVC, PP ou tout autre polymère courant), il est possible d'éliminer ce dernier par filtration ou tout autre moyen connu de l'homme de l'art.

En effet, les esters lactiques ne permettent pas la mise en solution des polymères précités pour les temps de traitement requis.

### 3. Le recyclage chimique du PLA

Après cette mise en solution, l'étape suivante consiste en la dépolymérisation du PLA afin de le ramener à son monomère de base (acide lactique) ou l'un de ses dérivés. Il est préférable de réaliser cette opération dans des conditions suffisamment douces afin d'éviter une dégradation de l'acide lactique ou de l'un de ses dérivés. Le fait de disposer du PLA mis en solution permet d'éviter de devoir impérativement dépasser sa température de fusion et donc de par les conditions plus douces, d'éviter ou de réduire les réactions de dégradation et de permettre ainsi d'obtenir un rendement proche de 100%.

La société demanderesse a aussi prouvé que la dégradation chimique du PLA pouvait être réalisée par hydrolyse à une température comprise entre 80 et 180°C, préférentiellement entre 100 et 150°C et plus préférentiellement entre 120 et 140°C, sous dépression ou un vide compris entre la pression atmosphérique et 10 bars voire plus. Cette étape d'hydrolyse permet la formation de l'acide lactique ou de l'un de ses sels. Elle est opérée par rupture d'un lien ester de la chaîne polymère suivie d'une attaque nucléophile. Cette attaque nucléophile peut être effectuée à l'aide d'eau ou d'une solution alcaline telle que NaOH, KOH,... La quantité d'eau ou de solution alcaline influençant la cinétique de réaction, il est néanmoins important de maintenir un compromis permettant d'éviter l'élimination d'une quantité trop importante d'eau lors des étapes de purification ultérieures. L'hydrolyse peut être réalisée avec utilisation ou non (réaction auto catalysée) d'un catalyseur acide de type acide de Lewis comme par exemple l'octoate d'étain, le lactate d'étain, l'octoate d'antimoine, l'octoate de zinc, l'APTS (acide para-toluène sulfonique), ou basique, de la famille des Guanidines, comme par exemple le TBD (triazabicyclodécène) et ses dérivés.

### 4. L'hydrolyse de l'ester d'acide lactique

Selon le procédé de l'invention, on peut également être envisagé d'hydrolyser l'ester d'acide lactique utilisé pour la mise en solution du PLA. Cette réaction est alors réalisée avec ajout ou non d'eau en présence ou non d'un catalyseur, fixé ou non sur une résine. Préférentiellement, celui-ci sera fixé. La quantité d'eau préconisée sera minimale pour un rendement maximal, afin de diminuer la dépense énergétique lors de la concentration de l'acide lactique obtenu. Cette hydrolyse peut être réalisée à pression atmosphérique ou sous dépression, elle peut également être menée en batch ou en continu par toutes méthodes connues de l'homme de l'art telle que la distillation réactive, l'utilisation d'un réacteur à écoulement piston, La réaction étant :

Ester d'acide lactique + Eau ⇄ Acide lactique + Alcool

Il est nécessaire d'effectuer l'extraction de l'alcool afin de déplacer l'équilibre de la réaction vers la formation de l'acide lactique.

L'acide lactique récupéré répond aux spécifications des applications industrielles ou autres du marché. Il pourra, dans certains cas, être utilisé pour reformer du PLA.

### 5. La purification de l'acide lactique obtenu par hydrolyse

Cette partie de l'invention consiste à la purification de l'acide lactique ou l'un des ses dérivés obtenu lors de l'hydrolyse du PLA, la pureté du produit pouvant être variable en fonction de l'utilisation visée. Il est possible d'atteindre des grades de haute qualité répondant aux critères du marché. Toutes techniques de purification connues de l'homme de l'art qui comprennent en général des étapes communes comme l'élimination des ions (résines d'échange ionique, extraction liquide/liquide, ), l'élimination de la couleur et autres impuretés (filtration, charbon actif, ), la concentration, la distillation (rectification, couche mince,) mais aussi la cristallisation, etc. peuvent être envisagées. L'hydrolyse étant réalisée à plus basse température, la dégradation du produit est moindre, ce qui facilite cette étape de purification.

De par ce procédé, il est donc possible de former une « boucle » PLA → acide lactique → PLA, impliquant une empreinte carbone plus faible que celle de la biomasse (biomasse → acide lactique → PLA → biomasse).

D'autres détails et particularités de l'invention, donnés ci-après à titre d'exemples non-limitatifs, ressortent de la description comme quelques formes possibles de sa réalisation.

### Exemple 1 : Recyclage de fibre de PLA par mise en solution dans un ester lactique suivie d'une hydrolyse

3,000 kg de fibres en PLA ont été broyés à l'aide d'un broyeur à couteaux. Ce broyat a ensuite été mis en solution dans 2,000 kg de LEt dans un réacteur vitrifié. La mise en solution a été réalisée à 130°C à pression atmosphérique. Une fois la mise en solution terminée, 2,250 kg d'eau déminéralisée ont été rajoutés. Le contenu du réacteur est alors chauffé jusqu'à l'obtention d'une pression de 2,4 bars. La température atteinte durant la réaction est de 137°C. Celle-ci restant inférieure à la température de fusion du PLA, évitant ainsi une dégradation de la matière. Les produits qui résultent de l'hydrolyse, ainsi que leur teneur respective sont repris dans le tableau 1. L'entièreté du PLA a été convertie en acide lactique. De façon surprenante, le lactate d'éthyle n'a été que légèrement hydrolysé.

**Tableau 1: Caractéristiques de l'hydrolysat**

| Acide lactique ^{(a)} (%) | Eau ^{(b)} (%) | LEt ^{(c)} (%) | Ethanol ^{(c)} (%) |
|---|---|---|---|
| 56,0 | 21,0 | 22,1 | 0,9 |

| | | | |
|---|---|---|---|
| (a) : déterminé par titrage (b) : déterminé par mesure Karl Fischer (c) : déterminés par GC lactate d'éthyle | | | |

L'acide lactique obtenu a été purifié par cristallisation. Dans un réacteur, 2 kg d'acide lactique ont été chauffés à 40°C sous agitation. Ensuite, 0.4 g d'une suspension contenant des cristaux ont été ajoutés. L'acide lactique est alors refroidi de 40 à 30°C en quelques heures. La suspension est ensuite centrifugée et les cristaux formés récupérés. La pureté du produit récupéré est de grade heat-stable.

Cette manière de procédé, nous permet en une seule étape réactionnelle et une purification simple, de récupérer 97% d'acide lactique que nous attendions théoriquement sur base du PLA initialement introduit.

### Exemple 2 : Mise en solution dans un ester lactique

Dans le cadre de cet exemple, du PLA broyé a été mis en solution dans différents esters d'acide lactique, le lactate de méthyle, le lactate d'éthyle et le lactate de n-butyle, à l'étuve à 130°C, à pression atmosphérique et sans agitation. Les résultats de ces mises en solution sont repris dans le tableau 2.

**Tableau 2 : Mise en solution de PLA dans différents esters d'acide lactique**

| test | Ester | Rapport massique PLA/ester | temps (h) | Mise en solution complète |
|---|---|---|---|---|
| 1 | L de méthyle | 1 | 1.5 | oui |
| 2 | L d'éthyle | 1 | 2 | oui |
| 3 | L de n-butyle | 1 | 3 | oui |

La solubilisation à pression atmosphérique du PLA dans les esters lactiques ou leurs alcools respectifs ont été comparée dans l'exemple suivant.

**Tableau 3: comparaison des mises en solution du PLA dans des esters lactiques ou leurs alcools respectifs.**

| test | Solvant | Rapport massique PLA/ester | Temp. (°C) | temps (h) | Mise en solution complète |
|---|---|---|---|---|---|
| 1 | Ethanol | 1 | 78°C | 3 | non |
| 2 | L d'éthyle | 1 | 120°C | 3 | oui |
| 3 | n-butanol | 1 | 120°C | 3 | non |
| 4 | L de n-butyle | 1 | 120°C | 3 | oui |

Dans le cas du lactate d'éthyle, différents rapports ester/PLA et différentes températures ont été étudiés et comparés après une durée de 4h sans agitation à pression atmosphérique. Les résultats sont repris dans le tableau 4.

**Tableau 4: Mise en solution dans du lactate d'éthyle de PLA broyés dans différentes proportions**

| test | Rapport massique PLA/LEt | t (°C) | Mise en solution à |
|---|---|---|---|
| 1 | 0,75 | 130 | 100% |
| 2 | 1 | 130 | 100% |
| 3 | 1,5 | 130 | 100% |
| 4 | 2 | 130 | 100% |
| 5 | 1 | 120 | 100% |
| 6 | 1,25 | 120 | 100% |
| 7 | 1,5 | 120 | 100% |
| 8 | 1,75 | 120 | ∼85% |
| 9 | 2 | 120 | ∼75% |

Les tests 8 et 9 ont été prolongés de 2 heures. L'entièreté du PLA du test 8 est dissoute. Par contre, 10% du PLA du test 9 n'ont pas été dissous.

Une mise en solution de fibres broyées (densité = 0,22) a été réalisée dans des conditions se rapprochant des conditions industrielles (agitation, quantités de matière plus importantes, à pression atmosphérique, ...). 1,5 kg de PLA ont été mis en solution dans 1 kg de lactate d'éthyle à 130°C. La fin de la mise en solution est observée 5 minutes après le dernier ajout. La solution obtenue avait une densité d'environ 1,25.

Il a également été tenté de mettre en solution différents polymères susceptibles de pouvoir contaminer le flux de PLA, dans le lactate d'éthyle, à 130°C, à pression atmosphérique, pendant 4h et sans agitation. Les résultats sont repris dans le tableau 5.

**Tableau 5 : Mise en solution dans du lactate d'éthyle de différents polymères broyés**

| Polymère | Rapport massique polymère/LEt | Mise en solution | Aspect du mélange |
|---|---|---|---|
| PEHD | 1 | non | suspension |
| PP | 0,14 | non | suspension |
| PET | 0,37 | non | suspension |
| PLA* | 1 | oui | solution |

| | | | |
|---|---|---|---|
| * donné à titre d'exemple comparatif | | | |

Ce dernier exemple tend à prouver la possibilité de séparer les polymères contaminant le PLA par une mise en solution dans un ester d'acide lactique. Pour le confirmer, des mises en solution dans du lactate d'éthyle, de PLA contaminé par l'un de ces polymères (10%) ont été réalisées à 130°C, pendant 4h et sans agitation (rapport massique polymère/LEt = 0,5). Les insolubles sont ensuite récupérés par filtration, puis lavés abondamment à l'eau, séchés et pesés. Les résultats sont repris dans le tableau 6. Les légères différences entre les masses avant et après tentative de mise en solution sont dues à la précision de la méthode utilisée.

**Tableau 6: Mise en solution dans du lactate d'éthyle de PLA contaminé par un autre polymère**

| Test | Polymère testé | Quantité de contaminant avant mise en solution | Quantité de contaminant récupéré |
|---|---|---|---|
| 1 | PEHD | 2,03 g | 2,04 g |
| 2 | PP | 1,99 g | 1,99 g |
| 3 | PET | 2,04 g | 2,03 g |

### Exemple 3 : Hydrolyse de PLA broyé en présence de NaOH

Dans un ballon de 2 litres sont ajoutés 600 g de PLA broyé et 400 g de lactate d'éthyle afin de mettre en solution le PLA. Ensuite, 938 g de NaOH 50% sont rajoutés par petites fractions au PLA dissous en évitant de monter à une température supérieure à 90°C. La réaction a durée 24h à pression atmosphérique. L'hydrolysat récupéré est ensuite filtré et analysé. La teneur en lactate de sodium étant de 64,5%, ce qui représente un rendement d'hydrolyse du PLA et du LEt de plus de 95%.

### Exemple 4 : Mise en solution de PLA broyé contaminé avec du polyéthylène téréphtalate (2%) dans du lactate d'éthyle suivie de la réaction d'hydrolyse en présence d'eau - Elimination du PET après la mise en solution

3,000 kg de gobelets en PLA broyés ont été contaminés par 2% de polyéthylène téréphtalate, soit 60 g. Le mélange de polymère a ensuite été mis en solution dans 2,000 kg de LEt dans un réacteur vitrifié. La mise en solution a été réalisée à 130°C, à pression atmosphérique et sous agitation. La solution a ensuite été filtrée à chaud afin de récupéré le PET non-dissous. Cette opération nous a permis de récupérer l'intégralité du polymère contaminant (soit 24 g).

Le filtrat a été transvasé dans un réacteur vitrifié et 2,250 kg d'eau déminéralisée ont été rajoutés. Le contenu du réacteur est alors chauffé jusqu'à l'obtention d'une pression de 2,4 bars. La température atteinte durant la réaction est de 135°C. Celle-ci restant inférieure aux températures de fusion du PLA. Le hydrolysat a été analysé et les résultats sont repris dans le tableau 7. L'entièreté du PLA a été convertie en acide lactique. Le lactate d'éthyle a été légèrement hydrolysé.

**Tableau 7: Caractéristiques de l'hydrolysat**

| Acide lactique ^{(a)} (%) | Eau ^{(b)} (%) | LEt ^{(c)} (%) | Ethanol ^{(c)} (%) |
|---|---|---|---|
| 54,2 | 20,6 | 23,4 | 1,8 |

| | | | |
|---|---|---|---|
| (a) : déterminé par titrage (b) : déterminé par mesure Karl Fischer (c) : déterminés par GC lactate d'éthyle | | | |

La solution obtenue a ensuite été concentrée par évaporation des composés volatils (éthanol, eau et lactate d'éthyle). L'acide lactique a ensuite été distillé. Cette manière de procédé, nous permet en une seule étape réactionnelle et une purification simple, de récupérer 97% d'acide lactique que nous attendions théoriquement sur base du PLA initialement introduit et de l'ester lactique partiellement hydrolysé.

### Exemple 5 : Mise en solution de PLA broyé contaminé avec du polypropylène (1%) dans du lactate d'éthyle suivie de la réaction d'hydrolyse en présence d'eau - Elimination du PP après réaction

3,000 kg de gobelets en PLA broyés ont été contaminés par 2% de polypropylène, soit 30 g. Le mélange de polymère a ensuite été mis en solution dans 2,000 kg de LEt dans un réacteur vitrifié. La mise en solution a été réalisée à 130°C à pression atmosphérique. Une fois la mise en solution terminée, 2,250 kg d'eau déminéralisée ont été rajoutés. Le contenu du réacteur est alors chauffé jusqu'à l'obtention d'une pression de 2,4 bars. La température atteinte durant la réaction est de 136°C. Celle-ci restant inférieure aux températures de fusion du PLA et du PP. L'hydrolysat récupéré est ensuite filtré. Les 30 g de polypropylène ont été récupérés intégralement. Le filtrat a été analysé et les résultats sont repris dans le tableau 8. L'entièreté du PLA a été convertie en acide lactique. Le lactate d'éthyle a été légèrement hydrolysé.

**Tableau 8: Caractéristiques de l'hydrolysat après filtration**

| Acide lactique ^{(a)} (%) | Eau ^{(b)} (%) | LEt ^{(c)} (%) | Ethanol ^{(c)} (%) |
|---|---|---|---|
| 55,9 | 19,9 | 22,1 | 2,1 |

| | | | |
|---|---|---|---|
| (a) : déterminé par titrage (b) : déterminé par mesure Karl Fischer (c) : déterminés par GC lactate d'éthyle | | | |

La solution obtenue a ensuite été concentrée par évaporation des composés volatils (éthanol, eau et lactate d'éthyle). L'acide lactique a ensuite été distillé. Cette manière de procédé, nous permet en une seule étape réactionnelle et une purification simple, de récupérer 98% d'acide lactique que nous attendions théoriquement sur base du PLA initialement introduit et de l'ester lactique partiellement hydrolysé.

## Revendications

1. Procédé de recyclage d'un mélange de polymères contenant nécessairement du PLA, **caractérisé en ce qu'**il comprend les étapes qui consistent à :
a. Broyer et/ou compacter le mélange de polymères jusqu'à obtenir un rapport poids/volume compris entre 0,05 et 1,4 t/m3
b. Mettre en solution le mélange de polymères broyés et/ou compactés dans un solvant du PLA afin de séparer le PLA des autres polymères
c. Récupérer les polymères non dissous pour traitement séparé et ultérieur
d. Récupérer la solution de PLA avec un rapport pondéral PLA/Solvant compris entre 0,5 et 3,0 et la soumettre à une réaction catalytique d'hydrolyse, à une température comprise entre 80 et 180°C et à une pression comprise entre 0,05 et 10 bars, afin de transformer le PLA en acide lactique ou un de ses dérivés
e. Purifier l'acide lactique ou son dérivé ainsi récupéré.

2. Procédé selon la revendication 1 **caractérisé en ce que** l'on effectue la mise en solution du mélange de polymères dans un ester lactique à une température préférentiellement comprise entre 80°C et la température d'ébullition de l'ester à la pression de service, pendant une période de temps suffisante pour obtenir un rapport pondéral PLA/ester lactique compris entre 0,5 et 3,0 et de préférence compris entre 0,75 et 2,0.

3. Procédé selon la revendication 2 **caractérisé en ce que** la pression de service est comprise entre 0,05 et 10 bars

4. Procédé selon la revendication 2 **caractérisé en ce que** l'ester lactique est choisi parmi les lactates d'alkyle dont le radical alkyle contient de 1 à 12 atomes de carbone

5. Procédé selon la revendication 4 **caractérisé en ce que** le lactate d'alkyle est choisi parmi le lactate de méthyle, d'éthyle, d'isopropyle, de butyle ou d'hexyle.

6. Procédé de recyclage du PLA par dépolymérisation de celui-ci en son monomère ou un de ses dérivés, comprenant le broyage et/ou le compactage du PLA, sa mise en solution dans un solvant, l'hydrolyse catalytique du PLA dissout en acide lactique ou un de ses dérivés, et la purification de l'acide lactique ou son dérivé ainsi récupéré, **caractérisé en ce que** l'on effectue la mise en solution du PLA dans un ester lactique à une température comprise préférentiellement entre 80°C et la température d'ébullition de l'ester lactique à la pression de service et ce pendant une période de temps suffisante pour obtenir un rapport pondéral PLA/ester lactique compris entre 0,5 et 3,0 et de préférence compris entre 0,75 et 2,0.

7. Procédé selon la revendication 6 **caractérisé en ce que** la pression de service est comprise entre 0,05 et 10 bars

8. Procédé selon la revendication 6 **caractérisé en ce que** l'ester lactique est choisi parmi les lactates d'alkyle dont le radical alkyle contient de 1 à 12 atomes de carbone

9. Procédé selon la revendication 8 **caractérisé en ce que** le lactate d'alkyle est choisi parmi le lactate de méthyle, d'éthyle d'isopropyle, de butyle ou d'hexyle.

## Patentansprüche

1. Verfahren zur Wiedergewinnung einer Mischung aus Polymeren, umfassend notwendigerweise PLA, **dadurch gekennzeichnet, dass** es die Schritte umfasst, die aus Folgendem bestehen:
a. Zerkleinern und/oder Kompaktieren der Mischung aus Polymeren, bis ein Verhältnis zwischen Gewicht und Volumen erhalten wird, das zwischen 0,05 und 1,4 t/m3 liegt,
b. Lösen der Mischung aus zerkleinerten und/oder kompaktierten Polymeren in einem Lösemittel der PLA, um die PLA von den anderen Polymeren zu trennen,
c. Wiedergewinnen der nicht gelösten Polymere für eine getrennte und spätere Behandlung,
d. Wiedergewinnen der Lösung aus PLA mit einem Gewichtsverhältnis zwischen PLA und Lösemittel, das zwischen 0,5 und 3,0 liegt, und Unterziehen dieser einer katalytischen Hydrolyse-Reaktion bei einer Temperatur zwischen 80 und 180 °C und bei einem Druck, der zwischen 0,05 und 10 bar liegt, um die PLA in Milchsäure und eines ihrer Derivate umzuwandeln,
e. Reinigen der Milchsäure oder seines so wiedergewonnenen Derivats.

2. Verfahren nach Anspruch 1, **dadurch gekennzeichnet, dass** die Lösung der Mischung aus Polymeren in einem Milchsäureester bei einer Temperatur, die vorzugsweise zwischen 80 °C und der Siedetemperatur des Esters beim Betriebsdruck liegt, während eines ausreichenden Zeitraums durchgeführt wird, um ein Gewichtsverhältnis zwischen PLA und Milchsäureester zu erhalten, das zwischen 0,5 und 3,0 und vorzugsweise zwischen 0,75 und 2,0 liegt.

3. Verfahren nach Anspruch 2, **dadurch gekennzeichnet, dass** der Betriebsdruck zwischen 0,05 und 10 bar liegt.

4. Verfahren nach Anspruch 2, **dadurch gekennzeichnet, dass** der Milchsäureester ausgewählt ist aus Alkyllaktaten, deren Alkylradikal zwischen 1 und 12 Kohlenstoffatomen umfasst.

5. Verfahren nach Anspruch 4, **dadurch gekennzeichnet, dass** das Alkyllaktat ausgewählt ist aus Methyl-, Ethyl-, Isopropyl-, Butyl- oder Hexyllaktat.

6. Verfahren zur Wiedergewinnung der PLA durch Depolymerisation dieser in ihr Monomer oder eines ihrer Derivate, umfassend die Zerkleinerung und/oder die Kompaktierung der PLA, ihre Lösung in einem Lösemittel, die katalytische Hydrolyse der gelösten PLA in Milchsäure oder eines ihrer Derivate, und die Reinigung der Milchsäure oder ihres so wiedergewonnenen Derivats, **dadurch gekennzeichnet, dass** die Lösung der PLA in einem Milchsäureester bei einer Temperatur, die vorzugsweise zwischen 80 °C und der Siedetemperatur des Milchsäureesters beim Betriebsdruck liegt, durchgeführt wird, und dies während eines ausreichenden Zeitraums, um ein Gewichtsverhältnis zwischen PLA und Milchsäureester zu erhalten, das zwischen 0,5 und 3,0 und vorzugsweise zwischen 0,75 und 2,0 liegt.

7. Verfahren nach Anspruch 6, **dadurch gekennzeichnet, dass** der Betriebsdruck zwischen 0,05 und 10 bar liegt.

8. Verfahren nach Anspruch 6, **dadurch gekennzeichnet, dass** der Milchsäureester ausgewählt ist aus den Alkyllaktaten, deren Alkylradikal von 1 bis 12 Kohlestoffatome umfasst.

9. Verfahren nach Anspruch 8, **dadurch gekennzeichnet, dass** das Alkyllaktat ausgewählt ist aus Methyl-, Ethyl-, Isopropyl-, Butyl- oder Hexyllaktat.

## Claims

1. Method for recycling a polymer blend necessarily containing PLA, **characterised in that** it comprises steps consisting of:
a. Grinding and/or compacting the polymer blend until a weight/volume ratio between 0.05 and 1.4 t/m3 is obtained
b. Placing the ground and/or compacted polymer blend in solution in a solvent of PLA in order to separate PLA from the other polymers
c. Retrieving the undissolved polymers for separate and subsequent processing
d. Retrieving the PLA solution with a PLA/Solvent ratio by weight between 0.5 and 3.0 and subjecting same to a catalytic hydrolysis reaction, at a temperature between 80 and 180°C and at a pressure between 0.05 and 10 bar, in order to convert PLA into lactic acid or any of the derivatives thereof
e. Purifying the lactic acid or derivative thereof retrieved.

2. Method according to claim 1 **characterised in that** the polymer blend is placed in solution in a lactic ester at a temperature preferentially between 80°C and the boiling point of the ester at working pressure, for a sufficient period of time to obtain a PLA/lactic ester ratio by weight between 0.5 and 3.0 and preferably between 0.75 and 2.0.

3. Method according to claim 2 **characterised in that** the working pressure is between 0.05 and 10 bar.

4. Method according to claim 2 **characterised in that** the lactic ester is chosen from alkyl lactates wherein the alkyl radical contains 1 to 12 carbon atoms.

5. Method according to claim 4 **characterised in that** the alkyl lactate is chosen from methyl, ethyl, isopropyl, butyl or hexyl lactate.

6. Method for recycling PLA by depolymerising same into the monomer thereof or any of the derivatives thereof, comprising grinding and/or compacting of the PLA, dissolution thereof in a solvent, catalytic hydrolysis of the dissolved PLA into lactic acid or any of the derivatives thereof, and purification of the lactic acid or derivative thereof retrieved, **characterised in that** the PLA is placed in solution in a lactic ester at a temperature preferentially between 80°C and the boiling point of the ester at working pressure, for a sufficient period of time to obtain a PLA/lactic ester ratio by weight between 0.5 and 3.0 and preferably between 0.75 and 2.0.

7. Method according to claim 6 **characterised in that** the working pressure is between 0.05 and 10 bar.

8. Method according to claim 6 **characterised in that** the lactic ester is chosen from alkyl lactates wherein the alkyl radical contains 1 to 12 carbon atoms.

9. Method according to claim 8 **characterised in that** the alkyl lactate is chosen from methyl, ethyl, isopropyl, butyl or hexyl lactate.
